# EUROPEAN PATENT APPLICATION

(11) **EP 2 974 677 A1**
(43) Date of publication of application: **20.01.2016**
(21) Application number: 14763791.2
(22) Date of filing: 21.01.2014
(51) Int. Cl.: A61B 17/16, A61B 17/17

(54) **OSTEOTOMY MARKER**

(30) Priority: 14.03.2013 ES 201330365
(71) Applicant: MOZO-GRAU, S.A., 47197 Valladolid (ES)
(72) Inventor: MOZO GRAU, Fernando, E-47197 Valladolid (ES)
(74) Representative: Pereira Toña, Maria Irache
(86) International application number: PCT/ES2014/070039
(87) International publication number: WO 2014/140395

(57) **Abstract**

An osteotomy marker (1) comprised of an interchangeable head (3) connected to a guide wire (2), so that the guide wire (2) is fixed to a surgical hand piece (15) and the interchangeable head (3) establishes a predetermined distance (D) with respect to a reference point at which that interchangeable head (3) guides a cutting tool (16) that performs the osteotomy; the interchangeable head (3) is formed by a central body (9) to which are joined a connector (5) and a support element (11) with a main body (28) that has two flat sides (29a, 29b) parallel to each other, establishing the predetermined distance (D) between the first flat side (29a) and a groove (10) in the central body (9), with the support element (11) resting on the first flat side (29a) at the reference point and the groove (10) marking the position and the predetermined distance (D) for performing the osteotomy.

## Description

### Object of the invention

This invention refers to a marker or guide for performing an osteotomy in bone surgery, and in this case, oral and maxillofacial surgery. It can be used in the medical-surgical field and in particular, in oral and maxillofacial implantology.

### Technical problem to be resolved and background of the invention.

An osteotomy is an excision of bone. That osteotomy must in many cases be performed in a specific situation or location, depending on the surgical needs of the case being treated.

Nowadays, most osteotomies are made on a "free-hand" basis, in this case depending on the skill of the surgeon or using a surgical splint comprised of a plastic piece custom-made for each patient that is placed in the area where the osteotomy is to be performed.

In osteotomies performed with the surgical splint, that splint must be made before the osteotomy is performed. In order to use it, first of all a scan must be made and then the splint must be made for the subsequent performing of the osteotomy, meaning that a splint is used for a patient which is then discarded, incurring a high cost and in many cases, that splint is not made prior to surgery, due to that important expense or due to a lack of planning. It may also occur that the surgical splint ordered cannot be used because the patient's anatomy has changed since the time when the scan was performed on the patient.

The object of this invention is an instrument that can be fitted to any patient, is reusable and can be applied in many surgical techniques; for instance traumas, maxillofacial surgery and dental implantology, so that this instrument can be used as a guide in performing an osteotomy on a patient, by being used to establish a pre-determined distance with respect to a reference point where the osteotomy is performed, thus using the instrument to avoid having to depend on the skill of the person responsible for performing the osteotomy.

### Description of the invention

The object of the invention is an osteotomy marker that is made up of a head connected to a guide wire; the guide wire is fixed to a surgical hand piece and the head is used to establish a pre-determined distance with respect to a reference point, to which a cutting tool connect to the surgical piece is guided by the head in performing the osteotomy.

The head of the osteotomy marker is formed by a central body which, in turn, has a groove, a support element consisting of a main body with two flat sides parallel to each other and a guide wire connector.

The support element and the connector are joined to the central body, establishing a predetermined distance between the first flat parallel side of the support element and the groove in the central body, so that the support element rests on the first flat parallel side at a reference point, and, through the groove, the central body marks the position and the predetermined distance at which the cutting tool will perform the osteotomy.

The support element is comprised of a lug and the connector is also comprised of a lug, so that the support element lug is inserted into a first recess of the central body and the connector lug is inserted into a second recess of the central body, thereby fastening the connector and the support element to the central body.

The osteotomy marker guide wire is comprised of a linear body with an appendage located at one end of that linear body.

The appendage located at the end of the guide wire linear body is formed by a cylindrical body with a central part that has a smaller diameter than the test of the cylindrical body and a pass-through hole into which one end of the guide wire is inserted.

The osteotomy marker and the head of the osteotomy marker are connected by the appendage of the guide wire and the head connector.

The linear body of the guide wire has two different parts; a first part, in which the marker is connected to the surgical hand piece and a second part, which has the appendage attached to one end of that part.
The head connector is formed by a hollow cylindrical body with a protrusion inside it.

When the head is connected to the guide wire, the inner protrusion of the connector is inserted into the central part of the guide wire appendage, and an O-ring is inserted between the appendage and the connector, to prevent the accidental dismantling of the marker.

### Description of the figures

To complete the description and to make it easier to understand the characteristics of the invention, a set of drawings is attached to this descriptive memorandum, as an inseparable part hereof, in which the following is shown, in an illustrative but non-exhaustive manner:
Figure 1 contains a perspective view of the marker forming the object of the invention, coupled to a surgical hand piece for working in one direction.
Figure 2 shows a perspective view of the marker forming the object of the invention coupled to a surgical hand piece for working in the opposite direction to that of figure 1.
Figure 3 shows an exploded view of the marker forming the object of the invention.
Figure 4 shows a view of the head of the marker forming the object of the invention, based on the use shown in figure 1.
Figure 5 shows a perspective view of the central body forming the object of the invention in which one side of the central body is visible.
Figure 6 shows a perspective view of the central body forming the object of the invention in which can be seen the side of the central body opposite the one seen in figure 5.
Figure 7 shows a perspective view of the marker head connector.
Figure 8 shows a perspective view of the marker head support element.
Figure 9 shows a plant view of the guide wire forming the object of the invention.
Figure 10 shows a perspective view of the appendage located at one end of the guide wire forming the object of the invention.
Figure 11 shows a perspective view of the clamp bracket used in the marker forming the object of the invention.

A list is given below of the different elements shown in the figures that make up the invention:
1.- marker,
2.- guide wire,
3.- head,
4.- appendage,
5.- connector,
6.- hollow cylindrical body,
7.- internal protrusion,
8.- cylindrical body,
9.- central body,
10.- groove,
11.- support element,
12.- first recess,
13.- second recess,
14.- clamp bracket,
15.- surgical hand piece
16.- cutting tool,
17a, 17b- lug,
18.- O-ring housing,
19.- pass-through hole,
20.- linear body,
21.-first part,
22.- second part,
23.- stop,
24.- pass-through opening,
25.- diametrical threaded hole,
26.- screw,
27.- O-ring,
28.- main body,
29a, 29b.- flat side,
D.- predetermined distance.

### Description of one way of using the invention

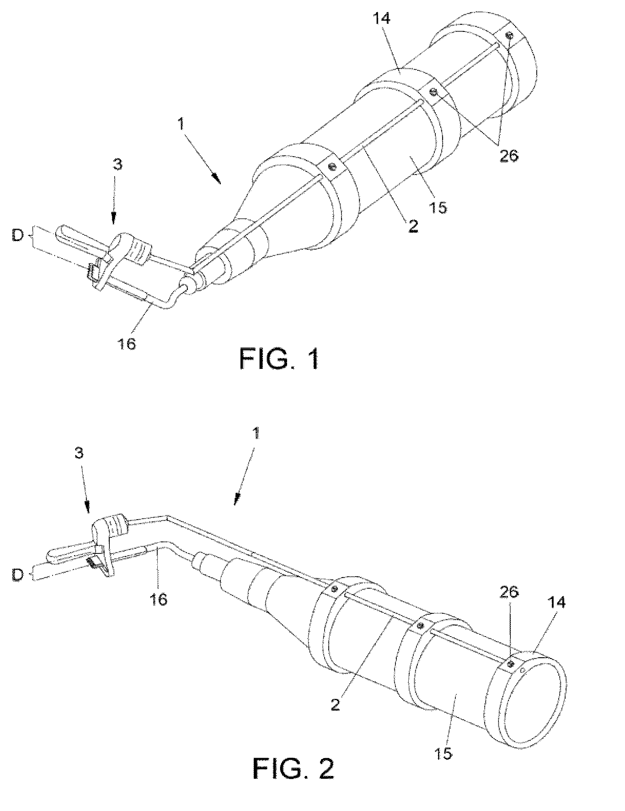

In view of the foregoing, and in reference to the numbering used in the figures, the object of the invention is a marker (1) (as shown in figures 1 and 2) used to perform osteotomies which marks a predetermined distance (D) with respect to the support point, and the surgical hand piece (15) performs the osteotomy without it being necessary to depend on elements other than the surgical instrument or the skill or expertise of the surgeon performing the osteotomy.

The marker (1) forming the object of the invention marks the predetermined distance (D) at which the osteotomy will be performed, providing control over the movements of the surgical hand piece (15) used to perform the osteotomy, with those movements being limited to actions within the predetermined distance (D) with respect to the support point, while allowing the movement of the marker hand piece assembly (15-1) in a lengthwise direction, thus performing the osteotomy and obtaining as a result the excision of the bone with a defined length and at a predetermined distance (D).

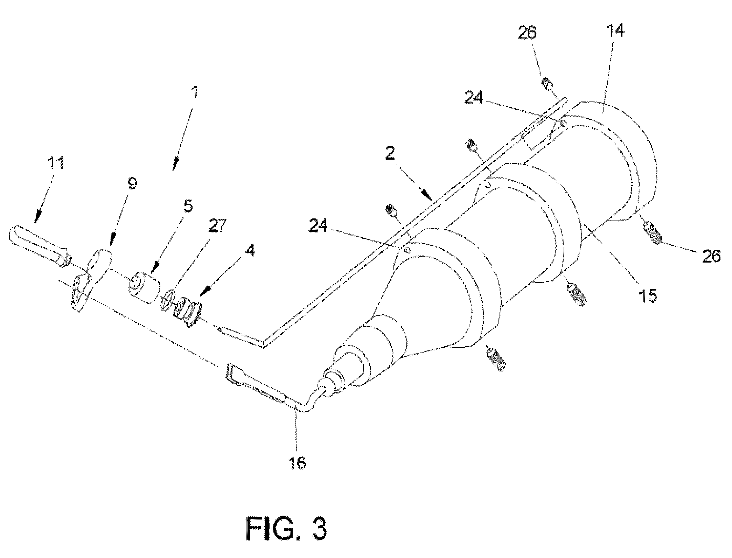

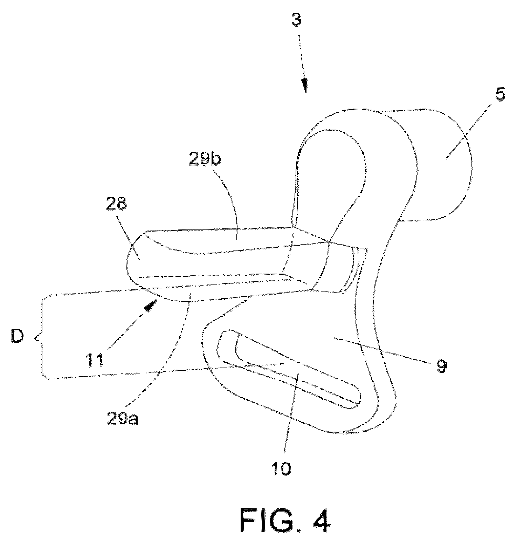

The marker (1) forming the object of the invention (as shown in figures 1, 2 and 3) is formed by a guide wire (2) connected to an interchangeable head (3). The interchangeable head (3) (shown in figure 4) has different sizes, depending on the predetermined distance "D" where the osteotomy is to be performed, so that osteotomies can be performed at predetermined distances (D) by simply changing the interchangeable head (3) in the marker (1) that is the object of the invention.

The marker (1) forming the object of the invention can be coupled to any surgical hand piece (15) by clamp brackets (14) that fasten together the surgical hand piece (15) and the guide wire (2) of the marker (1), thereby preventing relative movements between them.

The interchangeable head (3) (shown in figure 4) is formed by:
- a central body (9) which, in turn, has a groove (10) through which is inserted the cutting tool (16) of the surgical hand piece (15) used to perform the osteotomy;
- a support element (11) joined to the central body which has two parallel sides opposite each other, supporting the marker (1) on one of the above-described parallel sides;
- a connector (5) which is also joined to the central body (9), and is the coupling element between the interchangeable head (3) and the guide wire (2).

The predetermined distance (D) established by the marker for performing the osteotomy is defined by the interchangeable head (3) of the marker (1), and in the interchangeable head (3), the predetermined distance (D) is the distance between the opposite side of the support element (11) which provides the support at the reference point in the patient (bone or gum) and the groove (10) of the central body (9).

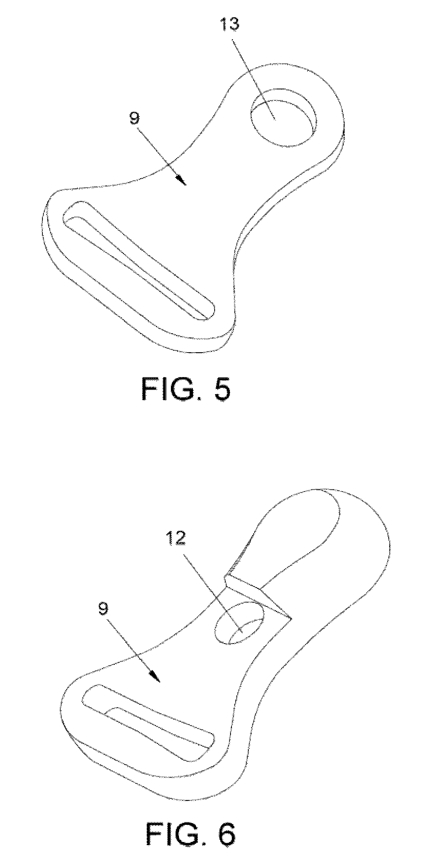

The central body (9) (shown in figures 5 and 6) is a flat polygonal-shaped part with curved sides, which has a first recess (12) to which the support element is fixed (11) and a second recess (13) to which the connect (5) is fixed. Both recesses (12,13) are on opposite sides of the central body (9).

When fixed to the central body (9), the connector (5) and the support element (11) are parallel to each other and perpendicular to the central body (9).

The fastening of the support element (11) and the connector (5) to the central body (9) in the first recess (12) and the second recess (13) respectively is obtained by friction, obtaining as a result the welding of the whole (5-9-11).

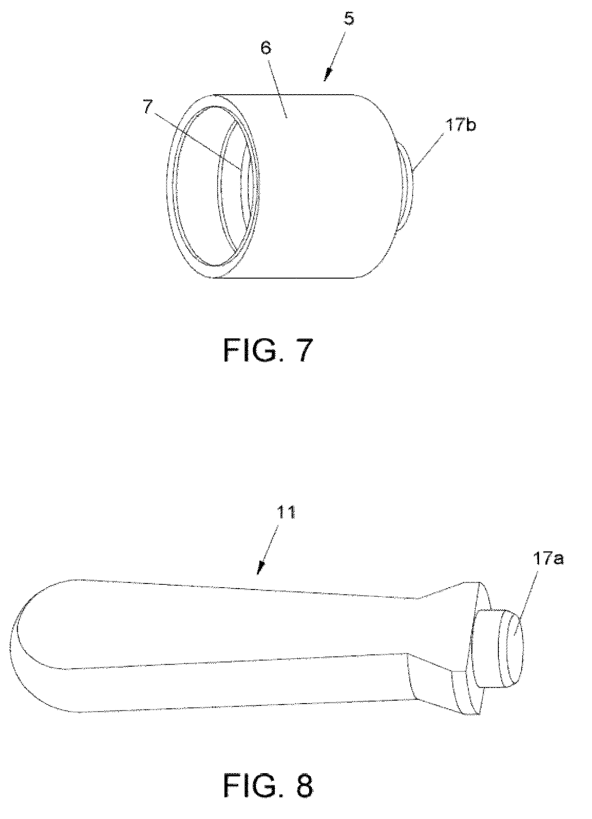

The shape of the groove (10) of the central body (9) is such that the section at one end is smaller than that of the opposite end, so that the cutting tool (16) is inserted through the end that is smaller and is moved through the groove (10) to the other end which is larger, thus permitting the cutting tool to move in a lateral and an anteroposterior direction without the thickness of the cutting tool (16) grazing the central body (9), and thereby preventing the cutting tool (16) from touching the central body (9), thus preventing its heating.
The support element (11) (shown in figure 8) has two flat sides (29a, 29b) which are parallel to each other, and it rests on the bone; this support point is used as a reference for performing the osteotomy at the predetermined distance (D). This predetermined distance (D) is the distance between the first flat side (29a) and the groove (10), and this predetermined distance (D) may vary, depending on the surgical needs of each technique. The support element (11) is formed by a lug (17a) at the end, through which it is joined to the central body (9); that lug (17a) fits into the first recess (12) of the central body (9), coupling the support element (11) to the central body (9) by friction, as described earlier.

The connector (5) (shown in figure 7) has a hollow cylindrical body (6) with an interior protrusion (7) and a lug (17b) at one end of the hollow cylindrical body (6), so that when the lug (17b) is inserted into the second recess (13) of the central body (9), the connector (5) is fixed to the central body (9).

The guide wire (2) is formed by a linear body (20), divided into a first part (21), through which the marker (1) is connected to the surgical hand piece (15), and a second part (22), at the end of which is the interchangeable head (3). Between the first part (21) and the second part (22) there is a change in the alignment of the linear body; in the preferential use of the invention, that change in alignment is 120° (this angle is not decisive, and it may be any angle, based on the shape of the surgical hand piece (15) and the cutting tool (16) of that surgical hand piece (15), to allow the cutting tool (16) to be positioned opposite the patient's bone). Furthermore, to fix the guide wire (2) to the interchangeable head (3), the guide wire (2) has an appendage (4) at the end to which the interchangeable head (3) is connected. The guide wire (2) is a flexible body, such that the flexible nature of that body allows the interchangeable head (3) to be moved by the cutting tool (16), as it goes deeper in cutting the bone.

The surgical hand piece (15) is the handle that holds the cutting tool (16), and inside it is a transmission system that transforms the rotating movements of the motor or the electric pulses of the ultrasonic motor into the movements required by the cutting tool (16) (cutter, reamer, ultrasonic insert) to produce the excision in the bone (osteotomy).

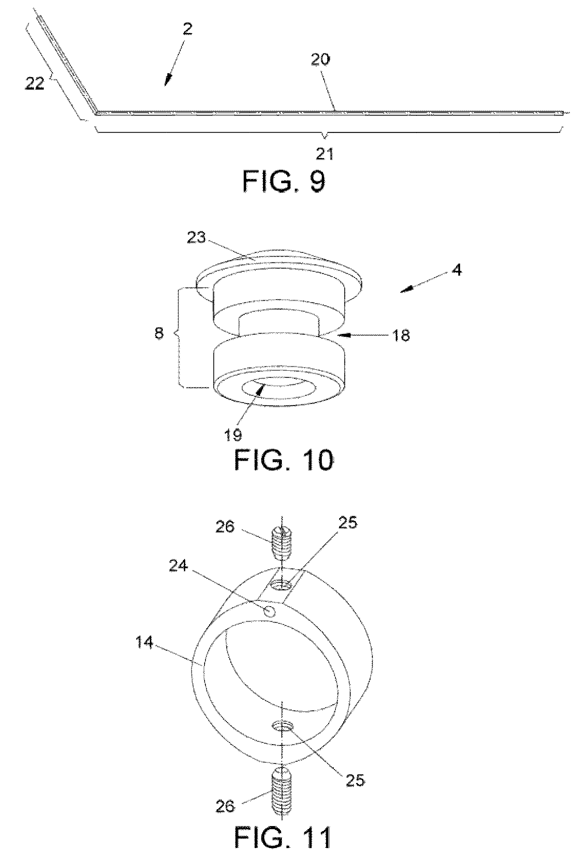

The appendage (4) (shown in figure 10) is formed by a cylindrical body (8) with a central part (18) that has a smaller diameter than the rest of the cylindrical body (8), and a pass-through hole (19) into which the guide wire (2) is inserted, and a tapered stop (23), at the opposite end to that of the connection with the guide wire (2). An O-ring (27) is housed in the central part (18) of the cylindrical body (8) with the smaller diameter). The connection of the appendage (4) to the guide wire (2) is executed by friction, after inserting the guide wire (2) into the pass-through hole (19) of the appendage (4), thus obtaining the welding of the appendage (4) to the guide wire (2).

The connection between the guide wire (2) and the interchangeable head (3) is executed by inserting the appendage (4) of the guide wire (2) into the connector (5) of the head, so that the cylindrical body (8) of the appendage remains inside the hollow cylindrical body (6) of the connector (5) of the head; this assembly can be dismantled by means of an O-ring (27) located in the central part (18). That O-ring (27) prevents vibrations and makes it impossible for the connector (5) to be accidentally dismantled.

The clamp brackets (14) (shown in figure 11) used to join the marker (1) to surgical hand piece (15), are formed by a cylindrical body with a pass-through hole on their perimeter (24) through which the guide wire (2) passes, with the surgical hand piece (15) surrounding the cylindrical body. Moreover, to secure the position of the guide wire (2) and fix the position of the clamp bracket on the surgical hand piece (15), each clamp bracket (14) has two diametrical threaded holes (25), into which screws (26) are inserted to limit the movement of the clamp bracket (14) with respect to the surgical hand piece (15) and the guide wire (2) with respect to the clamp bracket (14).

The invention should not be limited to the specific use decried in this document. Experts on the subject may develop other uses, in view of the descriptive given here. Consequently, the scope of the invention is defined by the following claims.

## Claims

1. An osteotomy marker (1) **characterised in that** it has an interchangeable head (3) connected to a guide wire (2), so that the guide wire (2) is fixed to a surgical hand piece (15) and the interchangeable head (3) establishes a predetermined distance (D) with respect to a reference point in which that interchangeable head (3) guides a cutting tool (16) connected to the surgical hand piece (15) in performing the osteotomy.

2. An osteotomy marker (1) as described in claim 1, **characterised in that** the interchangeable head (3) has:
- a central body (9) which in turn, has a groove (10),
- a support element (11), comprised of a central body (28) with two flat sides (29a, 29b) parallel to each other, and
- a connector (5) to the guide wire (2);
in such a way that the support element (11) and the connector (5) are joined to the central body (9), establishing a predetermined distance (D) between the first flat side (29a) of the support element (11) and the groove (10) of the central body (9), so that the support element (11) rests on the first flat parallel side (29a) at a reference point and the central body (9) marks the position and predetermined distance (D) at which the cutting tool (16) will perform the osteotomy by means of the groove (10).

3. An osteotomy marker (1), as described in claim 2, **characterised in that** the support element (11) is formed by lug (17a) and the connector (5) is also formed by a lug (17b), so that the support element (11) lug (17a) is inserted into a first recess (12) in the central body (9) and the connector (5) lug (17b) is inserted into a second recess (13) in the central body (9) thereby joining the connector (5) and the support element (11) to the central body (9).

4. An osteotomy marker (1) as described in any of the above claims, **characterised in that** the guide wire (2) is formed by a linear body (20) and an appendage (4) located at one end of that linear body (20).

5. An osteotomy marker (1) as described in claim 4, **characterised in that** the appendage (4) has:
- a cylindrical body (8) with a central part (18) that has a smaller diameter than the rest of the cylindrical body (8) and,
- a pass-through hole (19) into which one end of the guide wire is inserted (2).

6. An osteotomy marker (1) as described in claims 1 to 5, **characterised in that** the guide wire (2) and the interchangeable head (3) are joined through an appendage (4) of the guide wire (2) and the connector (5) of the interchangeable head (3).

7. An osteotomy marker (1) as described in claim 4, **characterised in that** the linear body (20) of the guide wire (2) is formed by two different parts; a first part (21) through which the connection between the marker (1) and the surgical hand piece (15) is executed and second part (22) in which the appendage (4) is located at one end of that second part (22).

8. An osteotomy marker (1) as described in claim 2, **characterised in that** the connector (5) is comprised of a hollow cylindrical body (6) with a protrusion inside it (7).

9. An osteotomy marker (1) as described in claims 4 to 6, **characterised in that** in the connection of the interchangeable head (3) and the guide wire (2), the internal protrusion (7) of the connector (5) is inserted into the central part (18) of the appendage (4) of the guide wire (2), and an O-ring (27) is inserted between the appendage (4) and the connector (5) to prevent the accidental dismantling of the marker (1).
